# EUROPEAN PATENT APPLICATION

(11) **EP 4 725 393 A1**
(43) Date of publication of application: **15.04.2026**
(21) Application number: 24818524.1
(22) Date of filing: 28.05.2024
(51) Int. Cl.: A61B 3/13, A61B 3/12, A61B 3/117, A61B 3/14

(54) **OPHTHALMIC OPERATING MICROSCOPE SYSTEM**

(30) Priority: 09.06.2023 CN 202310685380
(71) Applicant: Towardpi (Beijing) Medical Technology Ltd., Beijing 102206 (CN)
(72) Inventor: LU, Lirong, Beijing 102206 (CN); WANG, Xiao, Beijing 102206 (CN)
(74) Representative: Murgitroyd & Company
(86) International application number: PCT/CN2024/095670
(87) International publication number: WO 2024/250991

(57) **Abstract**

The present disclosure provides an ophthalmic surgical microscope system, including: a zoom module, a lens tube module and an eyepiece module arranged sequentially along a main optical axis from an object plane to an image plane, where: the lens tube module includes a lens tube and an optical path conversion device disposed on an end of the lens tube close to the zoom module; and the ophthalmic surgical microscope system includes an anterior segment optical path converter and a posterior segment optical path converter; and where, in a case of moving the anterior segment optical path converter into a main optical path, light reflected by the object plane propagates along a first direction to the anterior segment optical path converter after passing through the zoom module, and then propagates along a second direction through the lens tube to the eyepiece module after continuously reflected twice by the anterior segment optical path converter; and/or in a case of moving the posterior segment optical path converter into the main optical path, light reflected by the object plane propagates along the first direction to the posterior segment optical path converter after passing through the zoom module, and then propagates along the second direction through the lens tube to the eyepiece module after continuously reflected 3 times by the posterior segment optical path converter.

## Description

The present disclosure claims the priority from the CN patent application No. 202310685380.6 filed with the China National Intellectual Property Administration (CNIPA) on June 9, 2023, the contents of which are hereby incorporated by reference in their entirety.

### TECHNICAL FIELD

The present disclosure relates to the field of optical technologies and medical surgical microscopes, which can be applied to ophthalmic surgical scenarios.

### BACKGROUND

The eyeball can be divided into two segments, namely a front and a back segment. The eye tissue at the front segment of the eyeball is called anterior segment while the eye tissue at the back segment is called posterior segment. An ophthalmic surgical microscope typically has two observation modes, specifically: an anterior segment mode for allowing an anterior segment to be observed during an operation; and a posterior segment mode for allowing a posterior segment (i.e., a fundus) to be observed during an operation. Observing the posterior segment (i.e., observing the fundus) is different from observing the anterior segment, i.e., more optical elements are required for the posterior segment mode than the anterior segment mode.

### SUMMARY

The present disclosure provides an improved ophthalmic surgical microscope system which can attain an upright fundus imaging, without changing the operating space between the objective lens and the observed eyes, while accomplishing a miniaturized design of the ophthalmic surgical microscope. In addition, the upright fundus imaging solution provided in the embodiment of the present application can also reduce the number of light reflections, thereby reducing energy loss and lowering the processing requirements of the flip prism.

In the embodiments of the present disclosure, there is provided an ophthalmic surgical microscope system, comprising a zoom module, a lens tube module and an eyepiece module arranged sequentially along a main optical axis from an object plane to an image plane, wherein:
the lens tube module comprises a lens tube and an optical path conversion device disposed on an end of the lens tube close to the zoom module, the optical path conversion device comprising an anterior segment optical path converter and a posterior segment optical path converter; and in a case of moving the anterior segment optical path converter into a main optical path, light reflected by the object plane propagates along a first direction to the posterior segment optical path converter after passing through the zoom module, and then propagates along a second direction through the lens tube to the eyepiece module after continuously reflected twice by the anterior segment optical path converter; and/or in a case of moving the posterior segment optical path converter into the main optical path, light reflected by the object plane propagates along the first direction to the posterior segment optical path converter after passing through the zoom module, and then propagates along the second direction through the lens tube to the eyepiece module after continuously reflected 3 times by the posterior segment optical path converter.

### BRIEF DESCRIPTION OF THE DRAWINGS

Fig. 1 is a schematic diagram of an ophthalmic surgical microscope system provided by embodiments of the present disclosure;
Fig. 2 is a schematic diagram of an anterior segment optical path converter provided by embodiments of the present disclosure;
Fig. 3 is a schematic diagram of another anterior segment optical path converter provided by embodiments of the present disclosure;
Fig. 4 is a schematic diagram of a further anterior segment optical path converter provided by embodiments of the present disclosure;
Fig. 5 is a schematic diagram of a still further anterior segment optical path converter provided by embodiments of the present disclosure;
Fig. 6 is a schematic diagram of a posterior segment optical path converter provided by embodiments of the present disclosure;
Fig. 7 is a schematic diagram of another posterior segment optical path converter provided by embodiments of the present disclosure;
Fig. 8 is a schematic diagram of a further posterior segment optical path converter provided by embodiments of the present disclosure;
Fig. 9 is a schematic diagram of another ophthalmic surgical microscope system provided by embodiments of the present disclosure; and
Fig. 10 is a schematic diagram of another ophthalmic surgical microscope system provided by embodiments of the present disclosure

### DETAILED DESCRIPTION OF EMBODIMENTS

Reference now will be made to the drawings according to the embodiments of the present disclosure to fully describe the technical solution of the present disclosure through specific implementations. The embodiments depicted therein are only a part of the embodiments of the present disclosure.

In the posterior segment mode of the ophthalmic surgical microscope system, if provided without any interference, the additional optical element will cause the posterior segment image to be presented to an observer in a laterally inverted state, i.e., there will arise a laterally inverted intermediate image between the additional optical element and the objective lens of the ophthalmic surgical microscope. Based on the intermediate image, only an inverted fundus image with swapped left and right pupils of an observed person is captured through left and right eyepieces of the ophthalmic surgical microscope, which is not conducive to an ophthalmic operation.

In order to avoid presenting the observer with a laterally inverted image of the posterior segment and an image with swapped pupils, in some embodiments, the ophthalmic surgical microscope, when designed, can be provided with an optical element (also referred to as inverter module or flip prism) with functions of swapping optical paths and inverting an image, which can convert the intermediate image generated due to the arrangement of the additional optical element into an upright fundus image. In some embodiments, the inverter module can be sandwiched between the objective lens of the ophthalmic surgical microscope and the observed person, i.e., the inverter module is installed on the outside of the objective lens of the surgical microscope.

According to the design idea of the inverter, although the upright image of the posterior segment of the observed eye can be observed through the eyepieces of the ophthalmic surgical microscope, it is required to move or rotate the inverter module to the optical path of the surgical microscope between the objective lens of the surgical microscope and the observed eyes when the fundus is observed, and remove the inverter module from the optical path when the anterior segment is observed. For the technical solution, when the inverter module is introduced into the optical path, the working space between the objective lens of the surgical microscope and the observed eyes is reduced, which is not conducive to the ophthalmic operation; and in the anterior segment mode, in order to allow for the arrangement of the inverter module, the structure of the ophthalmic surgical microscope is prolonged, which hinders miniaturization of the ophthalmic surgical microscope.

To this end, the embodiments of the present disclosure provide an improved ophthalmic surgical microscope system which can attain an upright fundus image, without changing the operation space between the objective lens and the observed eyes, while accomplishing a miniaturized design of the ophthalmic surgical microscope.

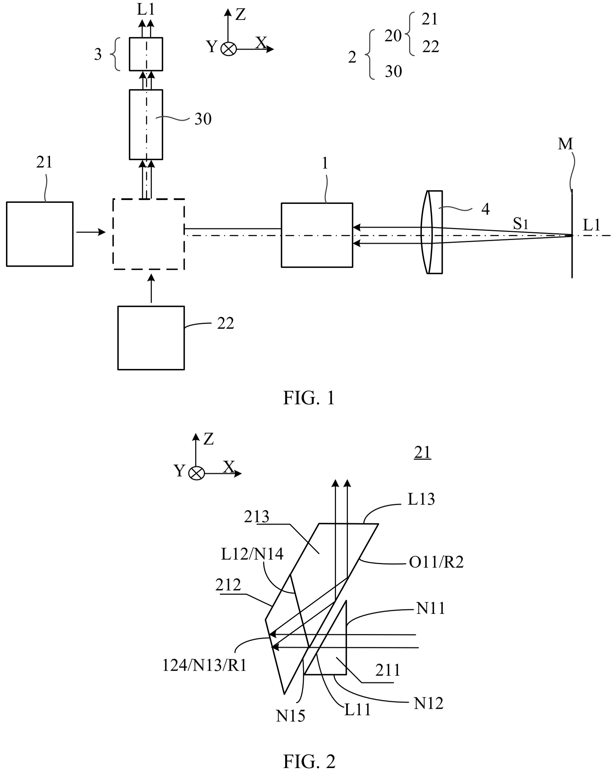

Fig. 1 illustrates a schematic diagram of an ophthalmic surgical microscope system provided by embodiments of the present disclosure.

As shown in Fig. 1, the surgical microscope system includes a zoom module 1, a lens tube module 2 and an eyepiece module 3 arranged sequentially along a main optical axis L1 from the object plane M to the image plane, where the lens tube module 2 includes a lens tube 30 and an optical path conversion device 20 that is disposed on an end of the lens tube 30 close to the zoom module 1, or on an end close to the zoom module 1 within the lens tube 30 (i.e., the optical path conversion device 20 may be disposed outside or inside the lens tube 30), and that includes an anterior segment optical path converter 21 and a posterior segment optical path converter 22.

In the embodiments of the present disclosure, the anterior segment optical path converter 21 and the posterior segment optical path converter 22 are two independent modules, and integrated in the internal structure of the surgical microscope, rather than arranged outside the objective lens 4 of the surgical microscope, thereby avoiding the impact on the operation space between the objective lens 4 of the surgical microscope and the observed eyes, which is conducive to the operation.

Moreover, in the anterior segment mode of the ophthalmic surgical microscope, it is required to move the posterior segment optical path converter 22 out of the main optical path of the microscope while moving the anterior segment optical path converter 21 into the main optical path of the microscope. On the contrary, in the posterior segment mode of the ophthalmic surgical microscope, it is required to move the anterior segment optical path converter 21 out of the main optical path of the microscope while moving the posterior segment optical path converter 22 into the main optical path of the microscope.

In this way, irrespective of the anterior segment mode or the posterior segment mode, only an optical path converter (the anterior segment optical path converter 21 or the posterior segment optical path converter 22) is disposed in the main optical path of the microscope while the other one is disposed outside the main optical path of the microscope. Therefore, this design solution will not prolong the structure of the surgical microscope, which is conducive to the miniaturization of the ophthalmic surgical microscope.

In the embodiments of the present disclosure, in the case of moving the posterior segment optical path converter 22 out of the main optical path while moving the anterior segment optical path converter 21 into the main optical path, light reflected from the object surface M propagates along a first direction (e.g., the X direction) to the anterior segment optical path converter 21 after passing through the zoom lens 1, and then propagates along a second direction (e.g., the Z direction) through the lens tube 30 to the eyepiece module 3 after continuously reflected twice by the anterior segment optical path converter 21.

Continuing with Fig. 1, by way of example, when the anterior segment is observed, the anterior segment optical path converter 21 can be moved along the X direction to the position indicated by the dotted box as shown therein. At this time, the anterior segment optical path converter 21 is moved into the main optical path, light S1 reflected by the observation object surface M propagates to the anterior segment optical path converter 21 after passing through the zoom module 1, and then propagates through the lens tube 30 to the eyepiece module 3 along the Z direction after continuously reflected twice by the anterior segment optical path converter 21. At this time, the observer can capture an upright stereoscopic image of the observed anterior segment.

In addition or alternatively, in the embodiments of the present disclosure, in the case of moving the anterior segment optical path converter 21 out of the main optical path while moving the posterior segment optical path converter 22 into the main optical path, light reflected by the observation object surface M propagates along the first direction (e.g., the X direction) to the posterior segment optical path converter 22 after passing through the zoom module 1, and then propagates along the second direction (e.g., the Z direction) through the lens tube 30 to the eyepiece module 3 after continuously reflected 3 times by the anterior segment optical path converter 22.

Continuing with Fig. 1, by way of example, when the posterior segment is observed, the anterior segment optical path converter 21 can be moved out of the position indicated by the dotted box as shown therein along the X direction, and the posterior segment optical path converter 22 then can be moved to the position indicated by the dotted box along the Z direction. At this time, the posterior segment optical path converter 22 is moved into the main optical path, and light S1 reflected by the observation object surface M propagates along the X direction to the posterior segment optical path converter 22 after passing through the zoom module 1, and propagates along the Z direction through the lens tube 30 to the eyepiece module 3 after continuously reflected 3 times by the posterior segment optical path 22. Accordingly, the observer can capture an upright stereoscopic image of the observed fundus.

In other embodiments, the anterior segment optical path converter 21 may also be configured as a prism or a prism combination where light is continuously reflected 4, 6 or more times; the posterior segment optical path converter 22 may also be configured as a prism or a prism combination where light is continuously reflected 5, 7 or more times. For the anterior segment optical path converter 21 and the posterior segment optical path converter 22, as the number of light reflections is increased, energy loss will be increased accordingly, and stray light interference will become more serious. With the optical path structure provided by the embodiments of the present disclosure, in the case of the same illumination intensity, the anterior segment optical path converter 21 continuously reflects light twice, and the posterior segment optical path converter 22 continuously reflects light three times, which can minimize the energy loss while improving imaging quality.

In addition, the more times the anterior segment optical path converter 21 and the posterior segment optical path converter 22 continuously reflect light, the higher the requirements for the processing and assembling process of the optical elements, which in turn results in a greater limitation to the development and manufacturing of precision optical instruments such as surgical microscopes, or even a difficult task. For example, although an optical path structure design can bring about a satisfactory simulation effect, the imaging effect of the finished product can barely be as good as the simulation effect of the optical path structure design due to the limitations of the processing and assembling technologies. However, in the embodiments of the present disclosure, the anterior segment optical path converter 21 is configured as an optical element where light is continuously reflected twice, and the posterior segment optical path converter 22 is configured as an optical element where light is continuously reflected three times. In addition to ensuring upright images of the anterior segment and the fundus, the present disclosure can reduce the number of light reflections, energy loss, stray light interference, requirements for the processing and assembling technologies for optical devices, and difficulties of the research and development and manufacturing of the ophthalmic surgical microscopes.

In the embodiments of the present disclosure, after the light reflected by the observed anterior segment is continuously reflected twice by the anterior segment optical path converter 21, the anterior segment image will not be displayed in a laterally inverted state, i.e., it can be ensured that an upright stereoscopic image of the anterior segment can be captured through the eyepiece module 3 of the surgical microscope.

In the embodiments of the present disclosure, after the light reflected by the observed anterior segment is continuously reflected three times by the posterior segment optical path converter 22, the posterior segment image will not be displayed in a laterally inverted state, nor will it contain swapped left and right pupil images, i.e., it can be ensured that an upright stereoscopic image of the posterior segment, without swapped left and right pupil images, can be captured through the eyepiece module 3 of the surgical microscope.

In the embodiments of the present disclosure, the ophthalmic surgical microscope includes two imaging optical paths for the left and the right eye, which are arranged symmetrically. Continuing with Fig. 1, the ophthalmic surgical microscope system further includes an objective lens 4 disposed on the optical path between the zoom module 1 and the observation object surface M. In addition, the ophthalmic surgical microscope system further includes other optical components, for example, a lighting module configured to provide illumination for the ophthalmic surgical microscope.

In an embodiment of the present disclosure, the first direction may be orthogonal to the second direction, where the first direction may be the X direction, and the second direction may be the Z direction. In other embodiments of the present disclosure, the first direction may not be orthogonal to the second direction. For example, the angle between the first direction and the second direction may be an acute angle, or may be an obtuse angle.

Figs. 2-5 are schematic diagrams of multiple types of ophthalmic optical path converters provided by the embodiments of the present disclosure.

As shown in Fig. 2, in an embodiment, the anterior segment optical path converter 21 may include a right triangle prism 211, a parallelogram prism 212, and an isosceles trapezoidal prism 213. The right triangle prism 211 includes a first right-angle surface N11, a second right-angle surface N12 and an inclined surface L11; the parallelogram prism 212 includes a first plane N13, a second plane N14 and a third plane N15, where the first plane N13 is parallel to the second plane N14; the isosceles trapezoidal prism 213 includes a first inclined surface L12, a second inclined surface L13 and a bottom surface O11. Wherein, the third plane N15 of the parallelogram prism 212 is coplanar with the bottom surface O11 of the isosceles trapezoidal prism 213, the second plane N14 of the parallelogram prism 212 is parallel to or fit with the first inclined surface L12 of the isosceles trapezoidal prism 213, the inclined surface L11 of the right triangle prism 211 is parallel to or fit with the third plane N15 of the parallelogram prism 212 and the bottom surface O11 of the isosceles trapezoidal prism 213, and the second right-angle surface N12 of the right triangle prism 211 is parallel to the second inclined surface L3 of the isosceles trapezoidal prism 213. The first right-angle surface N11 of the right triangle prism 211 is a light incident surface, the first plane N13 of the parallelogram prism 212 is a first reflective surface, the bottom surface O11 of the isosceles trapezoidal prism 213 is a second reflective surface, and the second inclined surface L13 of the isosceles trapezoidal prism 213 is a light outgoing surface.

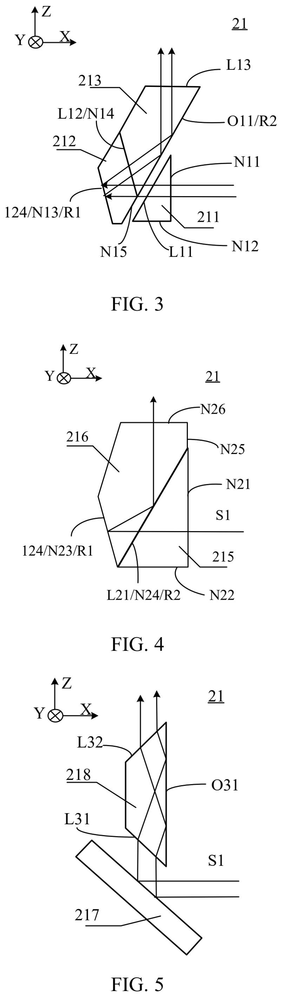

In the embodiments of the present disclosure, referring to Figs. 2 and 3, in order to facilitate assembling, for the parallelogram prism 212 as shown in Fig. 2, the prism angle formed between the third plane N15 and the first plane N13 can be cut, with the second right-angle surface N12 of the right triangle prism 211 being reference, such that the cut surface is located within the same plane as the second right-angle surface N12 of the right triangle prism 211. See Fig. 3 for the effect after cutting the angle.

Continuing with Figs. 2 and 3, in the embodiments of the present disclosure, the anterior segment optical path converter 21 is a combination of only 3 optical prisms, and 3 optical prisms have a regular shape, respectively, thereby saving efforts in designing of the optical structure while lowering the processing difficulty of optical components. In addition, in the embodiments of the present disclosure, the light is only reflected twice by the anterior segment optical path converter 21, and the anterior segment optical path converter 21 thus does not have the functions of swapping optical paths and flipping an image. Moreover, in the embodiments of the present disclosure, the anterior segment optical path converter 21 can adjust light propagating along the first direction (e.g., the X direction) as light propagating along the second direction (e.g., the Z direction), i.e., the anterior segment optical path converter 21 can deflect light propagation direction, which can function to adjust the light propagation direction. In this way, the present disclosure can reasonably adjust positions of the lens tube 30 and the eyepiece module 3, and reduce the overall size of the ophthalmic surgical microscope system.

Referring still to Figs. 2 and 3, in some embodiments of the present disclosure, the three prisms in the anterior segment optical path converter 21 may be prisms with the same refractive index. In the case of moving the anterior segment optical path converter 21 into the main optical path while moving the posterior segment optical path converter 22 out of the main optical path, light S1 reflected by the object surface M propagate along the X direction through the first right-angle surface N11 of the right triangle prism 211 to the anterior segment optical path converter 21 after passing through the zoom module 1, then undergoes total internal reflection at the bottom surface O11 of the isosceles trapezoidal prism 213 after reflected by the first plane N13 of the parallelogram prism 212, and finally outgoes along the Z direction and propagates through the lens tube 30 to the eyepiece module 3 after passing through the second inclined surface L13 of the isosceles trapezoidal prism 213.

In an embodiment of the present disclosure, in order to enable total internal reflection at the bottom surface O11 of the isosceles trapezoidal prism 213 in the anterior segment optical path converter 21, the isosceles trapezoidal prism 213 can be designed as a prism with a high refractive index which is greater than 1.7. According to the conditions for the total internal reflection, the critical angle of the total internal reflection θ₀=arcsin(n₀/n₁), where n₀ is the refractive index of the air, and n₁ is the refractive index of the isosceles trapezoidal prism 213.

In this way, when the incident angle of the light incident on the bottom surface O11 of the isosceles trapezoidal prism 213 (i.e., the second reflective surface R2 of the anterior segment optical path converter 21) is greater than the critical angle of the total internal reflection, the light propagates to the second inclined surface L13 of the isosceles trapezoidal prism 213 and then outgoes after undergoing total internal reflection at the second reflective surface R2.

In the embodiments of the present disclosure, by utilizing the principle of total internal reflection, there is no need to coat a reflective film over the second reflective surface R2, which can reduce the production costs while avoiding stray light interference.

Given the fact that the optical path length is equal to a product of multiplying the refractive index of the medium by the distance the light travels in the medium, the optical path length of light traveling in the prism increases with the refractive index of the prism. In the embodiments of the present disclosure, the greater the refractive index of the prism used in the anterior segment optical path converter 21, the greater the optical length of light traveling in the parallelogram prism 212 and the isosceles trapezoidal prism 213. In this way, in the case of keeping the total optical path of the surgical microscope system unchanged, the spatial volume of the zoom module 1 and the lens tube 30 can be compressed by increasing the refractive indices of the parallelogram prism 212 and the isosceles trapezoidal prism 213 in the anterior segment optical path converter 21, thus reducing the overall size of the ophthalmic surgical microscope system and finally achieving miniaturization of the ophthalmic surgical microscope system.

Alternatively, in other embodiments of the present disclosure, a film coating solution can be used to replace the total internal reflection solution. By way of example, continuing with Figs. 2 and 3, the outer side of the first plane N13 of the parallelogram prism 212 is coated with a film, and all or part of the outer side of the bottom surface O11 of the isosceles trapezoidal prism 213 is also coated with a film.

By way of example, continuing with Fig. 3, a film is coated over the outer side of the first plane N13 of the parallelogram prism 212, to form a reflective film 124, which can improve the reflection efficiency of the light S1 on the first reflective surface R1, thus avoiding stray light interference to the surgical microscope caused by light refraction while increasing microscope imaging brightness. Similarly, a film is coated over all or part of the bottom surface O11 of the isosceles trapezoidal prism 213, which can achieve a similar effect, and details thereof are omitted here for brevity.

Alternatively, in some other embodiments of the present disclosure, a solution with a combination of film coating and full internal reflection can also be employed. By way of example, still referring to Figs. 2 and 3, a film is coated over only the outer side of the first plane N13 of the parallelogram prism 212, not on the outer side of the bottom surface O11 of the isosceles trapezoidal prism 213. Wherein, it is required that an angle of light incident on the second reflective surface R2, after being incident on the anterior segment optical path converter 21 along the first direction and then reflected by the first reflective surface R1 in the anterior segment optical path converter 21, be greater than a critical angle when light is totally reflected on the second reflective surface R2.

By way of example, continuing with Fig. 3, a film is coated over the outer side of the first plane N13 of the parallelogram prism 212 to form a reflective film 124, and the bottom surface O11 of the isosceles trapezoidal prism 213 is not coated with a film but formed of a high refractive index transparent glass material with a refractive index greater than 1.7. In this way, it is required that the incident angle θ of light S1 incident on the second reflective surface R2, after being incident on the anterior segment optical path converter 21 along the X direction and then reflected by the first reflective surface R1 in the anterior segment optical path converter 21, be greater than a critical angle θ₀ when light is totally reflected on the second reflective surface R2, to thus ensure an occurrence of full internal reflection when light is incident on the second reflective surface R2, and achieve the reflection effect of the film coated over the second reflective surface R2.

In an embodiment of the present disclosure, referring to Fig. 4, the anterior segment optical path converter 21 includes a right triangle prism 215 and a pentaprism 216. The right triangle prism 215 includes a first right-angle surface N21, a second right-angle surface N22 and an inclined surface L21, and the pentaprism 216 includes a first plane N23, a second plane N24, a third plane N25 and a fourth plane N26 that is perpendicular to the third plane N25, where the second right-angle surface N22 of the right triangle prism 215 is parallel to the fourth plane N26 of the pentaprism 216, and the inclined surface L21 of the right triangle prism 215 is parallel to or fit with the second plane N24 of the pentaprism 216, and where the right-angle surface N21 of the right triangle prism 215 is a light incident surface, the first plane N23 of the pentaprism 216 is a first reflective surface R1, the second plane N24 of the pentaprism 216 is a second reflective surface R2, and the fourth plane N26 of the pentaprism 216 is a light outgoing surface.

In this way, the anterior segment optical path converter 21 is formed by a combination of only two optical prisms, and the two optical prisms have a regular shape, respectively, thereby saving efforts in designing of the optical structure while lowering the processing difficulty of optical components. In addition, in the embodiments of the present disclosure, the light is only reflected twice by the anterior segment optical path converter 21, and the anterior segment optical path converter 21 thus does not have the functions of swapping optical paths and flipping an image. Moreover, in the embodiments of the present disclosure, the anterior segment optical path converter 21 can adjust light propagating along the first direction (e.g., the X direction) as light propagating along the second direction (e.g., the Z direction), i.e., the anterior segment optical path converter 21 can deflect the light propagation direction, which can function to adjust the light propagation direction. In this way, the present disclosure can reasonably adjust positions of the lens tube 30 and the eyepiece module 3, and reduce the overall size of the ophthalmic surgical microscope system.

In an embodiment of the present disclosure, the pentaprism 216 can be formed of a material with a high refractive index greater than 1.7. According to the conditions for the total internal reflection, the critical angle of the total internal reflection θ₀=arcsin(n₀/n₂), where n₀ is the refractive index of the air, and n₂ is the refractive index of the pentaprism 216. When the incident angle of the light incident on the second plane N24 of the pentaprism 216 is greater than the critical angle of total internal reflection, the light propagates to the fourth plane N26 and then outgoes after undergoing the total internal reflection at the second reflective plane N24. In the embodiments of the present disclosure, by utilizing the principle of total internal reflection, there is no need to coat a reflective film over the second plane N24 of the pentaprism 216, which can reduce the production costs while avoiding stray light interference. In conjunction with the above description on the embodiments, the pentaprism 216 is formed of a material with a high reflective index, which can cause the spatial volume of the zoom module 1 and the lens tube module 2 to be compressed, thus reducing the overall size of the ophthalmic surgical microscope system and finally achieving miniaturization of the ophthalmic surgical microscope system.

Continuing with Fig. 4, when the anterior segment is observed, the anterior segment optical path converter 21 is moved into the main optical path, the light S1 reflected by the object surface propagates along the X direction to the first right-angle surface N21 and the inclined surface L21 of the right triangle prism 215 and the second plane N24 of the pentaprism 216, is then totally reflected on the second plane 24 of the pentaprism 216 after reflected by the first plane N23, and subsequently passes through the fourth plane N26 and propagates along the Z direction to the lens tube 30.

In a further embodiment of the present disclosure, still referring to Fig. 4, when the angle of the light incident on the second plane N24 of the pentaprism 216 is less than the critical angle of total internal reflection, the light cannot be totally reflected on the second plane N24, i.e., some of the light through the second plane N24 is lost, and the lost light continues to propagate in the system, which causes interference to the system.

To this end, in some embodiments, a film is coated over the outer side of the first plane N23 of the pentaprism 216, and a film is coated over a part of the outer side of the second plane N24 of the pentaprism 213. By way of example, continuing with Fig. 4, a reflective film 124 is coated over the outer side of the first plane N23 of the pentaprism 216, and a reflective film (not shown) is coated over a part of the outer side of the second plane N24 of the pentaprism 216. In this way, with the arrangement of the high reflective index film, light can be transmitted along the Z direction through the lens tube module 2 to the eyepiece module 3 after being reflected by the first plane N23 and the second plane N24 originally incapable of reflecting light and outgoing from the fourth plane N26, thereby improving the reflection efficiency of the light and increasing the image brightness; moreover, with the arrangement of the film, the present disclosure can also lower the requirement for the high reflective index material.

Alternatively, in other embodiments, a film is coated only over the outer side of the first plane N23 of the pentaprism 216, not over the outer side of the second plane N24 of the pentaprism 216. In the case, it is required that the angle of light S1 incident on the second reflective surface R2, after being incident on the anterior segment optical path converter 21 along the first direction (e.g.the X direction) and then reflected by the first reflective surface R1 in the anterior segment optical path converter 21, be greater than a critical angle when light is totally reflected on the second reflective surface R2. By way of example, still referring to Fig. 4, a film is coated only over the outer side of the first plane N13 of the pentaprism 216. In lieu of being coated with a film on the outer side of the second plane N24, the pentaprism 216 is formed of a material with a high reflective index greater than 1.7. In the case, it is required that the angle θ of light S1 incident on the second reflective surface R2, after being incident on the anterior segment optical path converter 21 along the X direction and then reflected by the first reflective surface R1 in the anterior segment optical path converter 21, be greater than a critical angle θ₀ when light is totally reflected on the second reflective surface R2. Therefore, with the arrangement of a coating with a high reflective index, the present disclosure can improve the reflection efficiency of the light and increase the image brightness; moreover, with the arrangement of the film, the present disclosure can also lower the requirement for the high reflective index material.

In an embodiment of the present disclosure, in conjunction with Fig. 5, the anterior segment optical path converter 21 may include a reflecting mirror 217 and a dove prism 218, where the reflecting mirror 217 is parallel to an inclined surface L31 of the dove prism 218. In the case, the light S incident on the anterior segment optical path converter 21 along the X direction outgoes from a further inclined surface L32 of the dove prism 218 after reflected by the first reflecting mirror 217, then passing through the inclined surface L31 of the dove prism 218 parallel to the reflecting mirror 217, and thereafter totally reflected by the bottom surface O31 of the dove prism 218.

The dove prism 218 is an image rotator. After light passes through the prism, the image will be inverted 180°, and the dove prism thus has the function of flipping an image. In addition, if the prism is rotated about its optical axis, the rotation angle of the image is twice greater than the rotation angle of the prism. As shown in Fig. 5, in an embodiment of the present disclosure, the dove prism 218 may be formed of a material with a high refractive material greater than 1.7. With reference to the related description on the above embodiments, the dove prism 218 with a high refractive index only has a one-prism structure, without the need for coating or other process, so that the light incident on the anterior segment optical path converter 21 along the X direction enters the dove prism 218 after reflected by the reflecting mirror 217, then continues to propagate after fully reflected by the bottom surface O31 thereof, and finally outgoes from the inclined surface L32 of the dove prism 218. With the dove prism 218, the anterior segment image can be inverted 180°, i.e., the anterior segment image is flipped 180°, in this embodiment.

In this way, in the case of keeping the total optical path of the surgical microscope system unchanged, the dove prism 218 with a high refractive index can also enable the spatial volume of the zoom module 1 and the lens tube 30 to be compressed, thus reducing the overall size of the ophthalmic surgical microscope system and finally achieving miniaturization of the ophthalmic surgical microscope system.

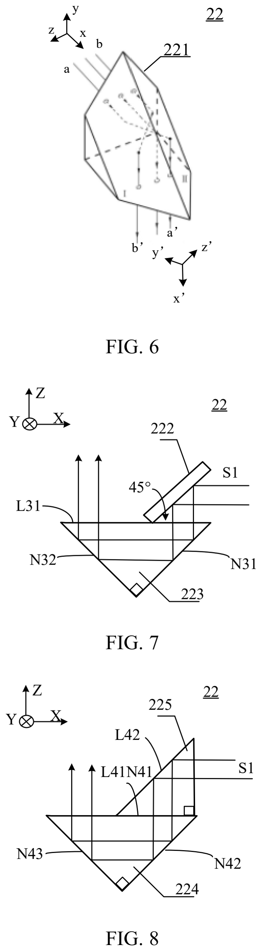

Figs. 6-8 illustrate schematic diagrams of multiple types of posterior segment optical path converters provided by embodiments of the present disclosure.

In an embodiment of the present disclosure, referring to Fig. 6, the posterior segment optical path converter 22 may include a roof prism 221. Two reflective surfaces of the roof prism that are perpendicular to each other are called roof surfaces, and a prism with roof surfaces are called roof prism.

In an embodiment of the present disclosure, referring to Fig. 6, light rays a and b in the coordinate system xyz enter vertically from one roof surface of the roof prism 221 and exit vertically from the other roof surface. The outgoing light rays are correspondingly marked as a' and b', and the coordinate system of the outgoing light rays is marked as x'y'z'. The propagation direction of the light rays a' and b' is deflected 90 degrees relative to that of the light rays a and b, and the propagation optical path of the light rays a' and b and that of the light rays a and b are spatially swapped, to thus accomplish functions of deflecting the optical path and flipping the image (swapping optical paths), i.e., the roof prism 221 has functions of deflecting an optical path and flipping an image.

In the embodiments of the present disclosure, the roof prism 221 may be formed of a material with a high refractive index that is, for example, greater than 1.7. In this way, in the case of keeping the total optical path of the surgical microscope system unchanged, the roof prism 221 with a high refractive index, as the anterior segment optical path converter, can enable the spatial volume of the zoom module 1 and the lens tube 30 to be compressed, thus reducing the overall size of the ophthalmic surgical microscope system and finally achieving miniaturization of the ophthalmic surgical microscope system.

In an embodiment of the present disclosure, referring to Fig. 7, the posterior segment optical path converter 22 may include a reflecting mirror 222 and a isosceles right-angle prism 223, where the reflecting mirror 222 is disposed at an angle of 45° relative to the bottom surface of the isosceles right-angle prism 223, and the light S1 incident on the posterior segment optical path converter 22 along the X direction is reflected by the reflecting mirror 222, then passes through the inclined surface L31 of the isosceles right-angle prism 223, and subsequently outgoes from the inclined surface L31 of the isosceles right-angle prism 223 after reflected sequentially by two right-angle surfaces (N31, N32) of the isosceles right-angle prism 223.

In this way, the posterior segment optical path converter 22 is formed by a combination of only two optical prisms, and the two optical prisms have a regular shape, respectively, thereby saving efforts in designing of the optical structure while lowering the processing difficulty of optical components. In addition, in the embodiments of the present disclosure, the light is only reflected 3 times by the posterior segment optical path converter 22, and the posterior segment optical path converter 22 thus have functions of swapping optical paths and flipping an image. Moreover, the light is reflected only 3 times by the posterior segment optical path converter 22, which can reduce energy loss as compared with the light being reflected by 5 times, 7 times or more times. In the embodiments of the present disclosure, the posterior segment optical path converter 22 can adjust light propagating along the first direction (e.g., the X direction) as light propagating along the second direction (e.g., the Z direction), i.e., the posterior segment optical path converter 22 can deflect a light propagation direction, which can function to adjust the light propagation direction. In this way, the present disclosure can reasonably adjust positions of the lens tube 30 and the eyepiece module 3, and reduce the overall size of the ophthalmic surgical microscope system.

In an embodiment of the present disclosure, the isosceles right-angle prism 223 may be formed of a material with a high refractive index greater than 1.7. In this way, in the case of keeping the total optical path of the surgical microscope system unchanged, the present disclosure can compress the spatial volume of the zoom module 1 and the lens tube 30, thus reducing the overall size of the ophthalmic surgical microscope system and finally achieving miniaturization of the ophthalmic surgical microscope system.

In the embodiments of the present disclosure, the isosceles right-angle prism 223 in Fig. 7 may be formed by splicing two small isosceles right-angle prisms of the same size. In an embodiment of the present disclosure, referring to Fig. 8, the posterior segment optical path converter 22 may include a first isosceles right-angle prism 224 and a second isosceles right-angle prism 225, where an inclined surface L41 of the first isosceles right-angle prism 224 is parallel to or fit with a right-angle surface N41 of the second isosceles right-angle prism 225, and light S1, which is incident on an inclined surface L42 of the second isosceles right-angle prism 224 in the posterior segment optical path converter 22 along the X ray, is reflected sequentially by the two right-angle surfaces (N42, N43) of the first isosceles right-angle prism 224 after reflected by the inclined L42 of the second isosceles right-angle prism 225, and then outgoes vertically from the inclined surface L41 of the first isosceles right-angle prism 224.

In this way, the posterior segment optical path converter 22 is a combination of two isosceles right-angle prisms of different sizes, which has functions of swapping optical paths and flipping an image. In an embodiment of the present disclosure, the optical prism (a first isosceles right-angle prism 224 and a second isosceles right-angle prism 225) is formed of a material with a high refractive index greater than 1.7. In the case of keeping the total optical path of the surgical microscope system unchanged, the spatial volume of the zoom module 1 and the lens tube 30 can be compressed, thus reducing the overall size of the ophthalmic surgical microscope system and finally achieving miniaturization of the ophthalmic surgical microscope system.

In the embodiments of the present disclosure, the isosceles right-angle prism 224 in Fig. 8 may be formed by splicing two small isosceles right-angle prisms of the same size.

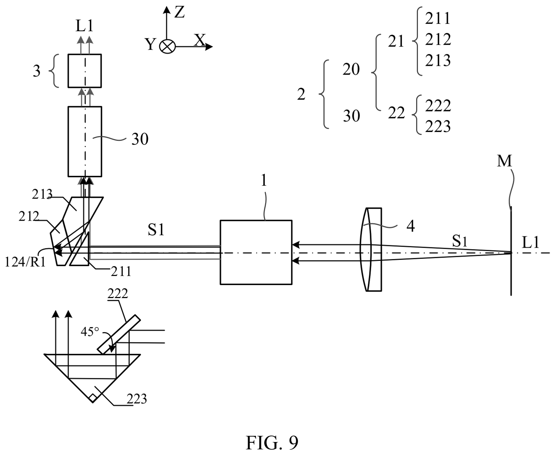

Fig. 9 is a schematic diagram of a further ophthalmic surgical microscope system provided by embodiments of the present disclosure.

In the embodiments of the present disclosure, referring to Fig. 9, the anterior segment optical path converter 21 is moved into or out of the main optical path along a first direction (e.g., the X direction), and the posterior segment optical path converter 22 is moved into or out of the main optical path along a second direction (e.g., the Z direction).

Alternatively, on the contrary, in other embodiments of the present disclosure, the anterior segment optical path converter 21 is moved into or out of the main optical path along the second direction (e.g., the Z direction), and the posterior segment optical path converter 22 is moved into or out of the main optical path along the first direction (e.g., the X direction).

In this way, irrespective of the anterior segment mode or the posterior mode, only one optical path converter (the anterior segment optical path converter 21 or the posterior segment optical path converter 22) is disposed in the main optical path of the microscope while the other one is disposed outside the main optical path of the microscope. Therefore, the design solution will not prolong the structure of the surgical microscope, which is conducive to the miniaturization of the ophthalmic surgical microscope.

In an embodiment of the present disclosure, referring to Figs. 3-9, the anterior segment optical path converter 21 in Figs. 3-5 can be arbitrarily matched with the posterior segment optical path converter 22 in Figs. 6-8 to form an optical path converter 20, and detailed examples will not be limited here. The structural combination pattern can meet the need of simultaneously observing the anterior segment and the fundus with only one ophthalmic surgical microscope. In addition, it is controlled to reflect light twice in the anterior segment optical path converter 21 and reflect light 3 times in the posterior segment optical path converter 22, thus avoiding energy loss resulting from excessive reflections. In the embodiments of the present disclosure, the anterior segment optical path converter 21 and the posterior segment optical path converter 22 are designed as a combination of 1-3 optical elements, rather than a separate irregular polygonal prism, which is helpful to reduce the optical processing requirements of the optical path conversion device. In the embodiments of the present disclosure, the optical path conversion device is integrated into the lens tube of the microscope and arranged close to the zoom module, which can avoid occupying the surgical space between the surgical microscope objective lens and the observed eyes, to facilitate the operation.

The features in the multiple embodiments of the present disclosure may be partly or fully coupled or combined with one another, and can cooperate with one another in various ways and be technically driven.

## Claims

1. An ophthalmic surgical microscope system, comprising a zoom module (1), a lens tube module (2) and an eyepiece module (3) arranged sequentially along a main optical axis (L1) from an object plane (M) to an image plane, wherein:
the lens tube module (2) comprises a lens tube (30) and an optical path conversion device (20) disposed on an end of the lens tube (30) close to the zoom module (1), the optical path conversion device (20) comprising an anterior segment optical path converter (21) and a posterior segment optical path converter (22); and
the ophthalmic surgical microscope system meets at least one of the following:
in a case of moving the anterior segment optical path converter (21) into a main optical path, light reflected by the object plane (M) propagates along a first direction to the anterior segment optical path converter (21) after passing through the zoom module (1), and then propagates along a second direction through the lens tube (30) to the eyepiece module (3) after being continuously reflected twice by the anterior segment optical path converter (21); and/or
in a case of moving the posterior segment optical path converter (22) into the main optical path, light reflected by the object plane (M) propagates along the first direction to the posterior segment optical path converter (22) after passing through the zoom module (1), and then propagates along the second direction through the lens tube (30) to the eyepiece module (3) after being continuously reflected 3 times by the posterior segment optical path converter (22).

2. The ophthalmic surgical microscope system of claim 1, wherein,
the anterior segment optical path converter (21) is moved into or out of the main optical path along the first direction, and the posterior segment optical path converter (22) is moved into or out of the main optical path along the second direction; or
the anterior segment optical path converter (21) is moved into or out of the main optical path along the second direction, and the posterior segment optical path converter (22) is moved into or out of the main optical path along the first direction.

3. The ophthalmic surgical microscope system of claim 1, wherein the first direction is orthogonal to the second direction.

4. The ophthalmic surgical microscope system of claim 1, wherein the anterior segment optical path converter (21) comprises: a right triangle prism (211), a parallelogram prism (212) and an isosceles trapezoidal prism (213), wherein,
the right triangle prism (211) comprises a first right-angle surface (N11), a second right-angle surface (N12) and an inclined surface (L11);
the parallelogram prism (212) comprises a first plane (N13), a second plane (N14) and a third plane (N15), the first plane (N13) being parallel to the second plane (N14); and
the isosceles trapezoidal prism (213) comprises a first inclined surface (L12), a second inclined surface (L13) and a bottom surface (O11); and
wherein,
the third plane (N15) of the parallelogram prism (212) is coplanar with the bottom surface (O11) of the isosceles trapezoidal prism (213), the second plane (N14) of the parallelogram prism (212) is parallel to or fit with the first inclined surface (L12) of the isosceles trapezoidal prism (213), the inclined surface (L11) of the right triangle prism (211) is parallel to or fit with the third plane (N15) of the parallelogram prism (212) and the bottom surface (O11) of the isosceles trapezoidal prism (213), and the second right-angle surface (N12) of the right triangle prism (211) is parallel to the second inclined surface (L13) of the isosceles trapezoidal prism (213); and
the first right-angle surface (N11) of the right triangle prism (211) is a light incident surface, the first plane (N13) of the parallelogram prism (212) is a first reflective surface (R1), the bottom surface (O11) of the isosceles trapezoidal prism (213) is a second reflective surface (R2), and the second inclined surface (L13) of the isosceles trapezoidal prism (213) is a light outgoing surface.

5. The ophthalmic surgical microscope system of claim 4, wherein,
an outer side of the first plane (N13) of the parallelogram prism (212) is coated with a film, and all or a part of an outer side of the bottom surface (O11) of the isosceles trapezoidal prism (213) is coated with a film; or
only the outer side of the first plane (N13) of the parallelogram prism (212) is coated with a film while the outer side of the bottom surface (O11) of the isosceles trapezoidal prism (213) is not coated with a film, wherein an angle of light incident on the second reflective surface (R2), after being incident on the anterior segment optical path converter (21) along the first direction and then reflected by the first reflective surface (R1) in the anterior segment optical path converter (21), is required to be greater than a critical angle when light is totally reflected on the second reflective surface (R2).

6. The ophthalmic surgical microscope system of claim 4, wherein the isosceles trapezoidal prism is formed of a material with a refractive index greater than 1.7.

7. The ophthalmic surgical microscope system of claim 1, wherein the anterior segment optical path converter (21) comprises: a right triangle prism (215) and a pentaprism (216), wherein,
the right triangle prism (215) comprises a first right-angle surface (N21), a second right-angle surface (N22) and an inclined surface (L21); and
the pentaprism (216) comprises a first plane (N23), a second plane (N24), a third plane (N25) and a fourth plane (N26), the third plane (N25) being perpendicular to the fourth plane (N26);
wherein,
the second right-angle surface (N22) of the right triangle prism (215) is parallel to the fourth plane (N26) of the pentaprism (216), and the inclined surface (L21) of the right triangle prism (215) is parallel to or fit with the second plane (N24) of the pentaprism (216); and
the first right-angle surface (N21) of the right triangle prism (215) is a light incident surface, the first plane (N23) of the pentaprism (216) is a first reflective surface (R1), the second plane (N24) of the pentaprism (216) is a second reflective surface (R2), and the fourth plane (N26) of the pentaprism (216) is a light outgoing surface.

8. The ophthalmic surgical microscope system of claim 7, wherein,
an outer side of the first plane (N23) of the pentaprism (216) is coated with a film, and a part of an outer side of the second plane (N24) of the pentaprism (216) is coated with a film; or
only the outer side of the first plane (N23) of the pentaprism (216) is coated with a film while the outer side of the second plane (N24) of the pentaprism (216) is not coated with a film, wherein an angle of light incident on the second reflective surface (R2), after being incident on the anterior segment optical path converter (21) along the first direction and then reflected by the first reflective surface (R1) in the anterior segment optical path converter (21), is required to be greater than a critical angle when light is totally reflected on the second reflective surface (R2).

9. The ophthalmic surgical microscope system of claim 1, wherein the anterior segment optical path converter (21) comprises: a dove prism (218) and a reflecting mirror (217), wherein,
the reflecting mirror (217) is parallel to an inclined surface of the dove prism (218); and
light incident on the anterior segment optical path converter (21) along the first direction outgoes from a further inclined surface (L32) of the dove prism (218) after being reflected by the reflecting mirror (217), then passing through an inclined surface (L31) of the dove prism (218) parallel to the reflecting mirror, and thereafter being totally reflected by the bottom surface (O31) of the dove prism (218).

10. The ophthalmic surgical microscope system of claim 1, wherein,
the posterior segment optical path converter (22) comprises: a isosceles right-angle prism (223) and a reflecting mirror (222), wherein the reflecting mirror (222) is disposed at an angle of 45° relative to the bottom surface of the isosceles right-angle prism (223), and light incident on the posterior segment optical path converter (22) along the first direction is reflected by the reflecting mirror (222), then passes through an inclined surface (L31) of the isosceles right-angle prism (223), and subsequently outgoes from the inclined surface (L31) of the isosceles right-angle prism (223) after reflected sequentially by two right-angle surfaces (N31, N32) of the isosceles right-angle prism (223); or
the posterior segment optical path converter (22) comprises: a first isosceles right-angle prism (224) and a second isosceles right-angle prism (225), wherein an inclined surface (L41) of the first isosceles right-angle prism (224) is parallel to or fit with a right-angle surface (N41) of the second isosceles right-angle prism (225), and light incident on the posterior segment optical path converter (22) along the first direction is reflected sequentially by two right-angle surfaces (N42, N43) of the first isosceles right-angle prism (224) after reflected by an inclined surface (N42) of the second isosceles right-angle prism (225), and then outgoes from an inclined surface (L41) of the first isosceles right-angle prism (224).
